# EUROPEAN PATENT APPLICATION

(11) **EP 2 130 928 A1**
(43) Date of publication of application: **09.12.2009**
(21) Application number: 08104225.1
(22) Date of filing: 02.06.2008
(51) Int. Cl.: C12Q 1/68

(54) **Nucleic acids and methods for detecting turfgrass pathogenic fungi**

(71) Applicant: Omya Development AG, 4665 Oftringen (CH)
(72) Inventor: Di Maiuta, Nicola, 4528 Zuchwil (CH); Schwarzentruber, Patrick, 8113 Boppelsen (CH)
(74) Representative: Domenego, Bertrand

(57) **Abstract**

The present invention relates to the use of at least one nucleic acid comprising or consisting of:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
R represents A or G
Y represents C or T
M represents A or C
W represents A or T
H represents A or C or T

(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii);for the detection of nucleic acids from one or more fungi in a sample.

## Description

### Field of the invention

The present invention relates to nucleic acids and methods for detecting organic substrate pathogenic fungi.

### Background of the invention

Disease in lawngrasses or turfgrasses develops from an interaction among a susceptible plant, an environment favorable for disease development, and a pathogenic organism, usually a fungus. Such fungi may also develop on decorative grasses, plants and crops; indeed, they may appear on any suitable organic substrate. Thus, treatment of a diseased substrate, especially turfgrass, usually consists in applying fungicides that will either kill the fungus or keep it from growing.

However, the first step in disease management, and especially turfgrass disease management, should always consist in identifying the causative pathogenic agent. Indeed, it is important to have identified the disease correctly, so that an appropriate fungicide can be selected. Arbitary selection and application of fungicides without knowledge of the disease cause can do as much harm as good. Using the wrong fungicide wastes money and may involve the risk of exacerbating the disease, as well as causing other unwanted side effects.

Classical methods for the identification of the causative pathogenic agent essentially rely on the symptoms which can be observed on the individual plant and on the turf stand, as well as on the fungal structures, such as mycelia or spores, which can be found in the vicinity of the diseased turfgrass.

However, these methods may require a long time to be implemented, since they often involve the isolation and the culture of the fungi in a laboratory. Besides, differentiating closely related fungal species can prove difficult.

Accordingly, molecular biology methods have been developped which circumvent these difficulties. One of the most popular fungal detection methods relies on the PCR amplification of the internal transcribed spacers (1, 2) and the 5.8S rRNA gene (ITS1-5.8S-ITS2) from the fungal rRNA operon (Goodwin et al. (1995) Plant Pathology 44:384-391; Ranjard et al. (2001) Applied and Environmental Microbiology 67:4479-4487). However, a specific primer pair is often necessary for the indentification of a given fungal species, which renders this method cumbersome where the identity of pathogenic fungus is unknown and is sought for. Furthermore, some fungal species can not be differentiated using the primers currently available which target this region. Accordingly, this method is not used in routine for determining the antifungal agent most adapted to treat a given turfgrass disease.

It is therefore an object of the invention to provide a method which allows the rapid specific detection of nucleic acids from the fungi most commonly involved in turfgrass diseases.

### Summary of the invention

The present invention arises from the identification, by the inventors, of a conserved region within the rRNA operon of the genome of pathogenic fungi generally affecting turfgrasses, but also possibly other organic substrates, which is liable to be used as a target in the frame of nucleic acid amplification-based detection method of most of such pathogenic fungi.

Thus the present invention relates to the use of at least one nucleic acid comprising or consisting of:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
   R represents A or G
   Y represents C or T
   M represents A or C
   W represents A or T
   H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii); for the detection of nucleic acids from one or more fungi in a sample.

In a preferred embodiment, the present invention more particularly relates to the use of at least one nucleic acid comprising or consisting of:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii); for the detection of nucleic acids from one or more fungi in a sample.

The present invention also relates to a method for detecting nucleic acids from one or more fungi in a sample, wherein at least one nucleic acid comprising or consisting of:
(i) 
[?]
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
   R represents A or G
   Y represents C or T
   M represents A or C
   W represents A or T
   H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii);
is contacted with the sample.

In a preferred embodiment, the invention more particularly relates to a method for detecting nucleic acids from one or more fungi in a sample, wherein at least one nucleic acid comprising or consisting of:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii); is contacted with the sample.

The present invention also relates to a method for treating a diseased organic substrate, which comprises the steps of:
a) detecting the absence or the presence of nucleic acids from at least one pathogenic fungus in a sample of the substrate, with at least one nucleic acid according to the invention as defined above;
b) if nucleic acids from one or more pathogenic fungi have been detected in step a), selecting one or more antifungal agents which target the one or more pathogenic fungi from which nucleic acids have been detected;
c) applying the selected one or more antifungal agents of step b) to the diseased substrate.

The present invention also relates to a kit for the detection of fungi, comprising each one of the nucleic acids represented by:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
   R represents A or G
   Y represents C or T
   M represents A or C
   W represents A or T
   H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii).

In a preferred embodiment, the invention more particularly relates to a kit for the detection of fungi, comprising each one of the nucleic acids represented by:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii).

The present invention also relates to a nucleic acid comprising or consisting of:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
   R represents A or G
   Y represents C or T
   M represents A or C
   W represents A or T
   H represents A or C or T
(ii) a portion of SEQ ID NO: 1, provided the said nucleic acid binds under stringent conditions to a nucleic acid comprising or consisting of the complementary sequence of SEQ ID NO: 1, or
(iii) complementary sequences of (i) and (ii).

In a preferred embodiment, the invention more particularly relates to a nucleic acid comprising or consisting of:
(i) GTGARTCATCGAAWYTTTGAACGCA (SEQ ID NO: 2), wherein:
   R represents A or G
   Y represents C or T
   W represents A or T
(ii) a portion of SEQ ID NO: 2, provided said nucleic acid binds under stringent conditions to a nucleic acid comprising or consisting of the complementary sequence of SEQ ID NO: 1, or complementary sequences of (i) and (ii).

In another preferred embodiment, the invention more particularly relates to a nucleic acid as defined above, comprising or consisting of a sequence selected from the group consisting of SEQ ID NO: 1 to 38, or complementary sequences thereof.

### Detailed description of the invention

Nucleic acids as intended herein can be of any type, however it is preferred that they be DNA.

"Stringent conditions" can be easily be defined by the man skilled in the art using common knowledge. If necessary, guidance for defining such conditions can be found in numerous textbooks, such as Tijssen, 1993, Laboratory Techniques in Biochemistry and Molecular Biology - Hybridization with Nucleic Acid Probes, Part I, Chapter 2 "Overview of principles of hybridization and the strategy of nucleic acid probe assays", Elsevier, New York. Preferably, stringent conditions according to the invention are constituted of an annealing temperature of 60°C carried out in a PCR reaction medium comprising, *e*.*g*. 50 mM KCl, 1.5 mM MgCl₂ and 10 mM Tris pH 8.3.

As intended herein a "portion" of nucleic acid preferably comprises a number of nucleotides sufficient to provide for a specific hybridisation to the nucleic acid comprising or consisting of the complementary sequence of SEQ ID NO: 1. Thus, the portion of nucleic acid preferably comprises at least 9 nucleotides, more preferably at least 15 nucleotides, even more preferably at least 20 nucleotides, and most preferably at least 25 nucleotides.

As intended herein, it is preferred that the maximum length of the nucleic acids according to the invention is less than 500 nucleotides.

Preferably, the at least one nucleic acid as defined above is used as a primer in a nucleic acid amplification-based detection method, such as Amplification Fragment Length Polymorphism (AFLP) or Terminal Restriction Fragment Length Polymorphism (TRFLP or T-RFLP).

Nucleic acid amplification-based detection methods are particularly well known to one of skill in the art. Terminal Restriction Fragment Length Polymorphism (TRFLP or T-RFLP), for instance, is notably described by Formey et al. (1997) Applied and Environmental Microbiology 63:4516-4522.

Where the at least one nucleic acid as defined above is used as a primer, it is preferably used in association with at least one other primer. The other primer can be any primer targeting a sequence comprised within the genome of the one or more fungi. However, it is preferred that the other primer targets the 18S rDNA/ITS1 region or the ITS2/28S rDNA region of the one or more fungi.

The fungal rRNA operon comprises the 18S rRNA gene, the Internal Transcribed Spacer 1 (ITS1), the 5.8S rRNA gene, the Internal Transcribed Spacer 2 (ITS2), and the 28S rRNA gene. The 18S rDNA/ITS1 and ITS2/28S rDNA regions thus relate to the regions of the genome of the one or more fungi in the vicinity respectively of the junction of the 18S rRNA gene and ITS1, and of the junction of TIS2 and the 28S rRNA gene. As will be clear to anyone of skill in the art, where the at least one nucleic acid as defined above is used as a forward primer, the primer which targets the 18S rDNA/ITS1 region or the ITS2/28S rDNA region will be a reverse primer, and *vice versa.* The sequences of the 18S rDNA/ITS1 and ITS2/28S rDNA regions are well known to one of skill in the art and can usually be accessed from public sequence databases. Where sequences of the 18S rDNA/ITS1 and ITS2/28S rDNA regions would not be publicly available for a particular fungus species, they can be routinely sequenced. Besides, it is well within the common knowledge of anyone of skill in the art to select primers within the known sequences.

By way of example of a primer targeting the 18S rDNA/ITS1 region, one can cite the so-called "ITS1-F primer" of sequence TCCGTAGGTGAACCTGCGG (SEQ ID NO: 39). Conversely, by way of example of a primer targeting the ITS2/28S rDNA region one can cite the so-called "ITS4 primer" of sequence TCCTCCGCTTATTGATATGC (SEQ ID NO: 40). Other examples of primers liable to be used with the primers according to the invention include the "ITS5 primer" of sequence GGAAGTAAAAGTCGTAACAAGG (SEQ ID NO: 41) and the "SR6R primer" of sequence AAGWAAAAGTCGTAACAAGG (SEQ ID NO: 42). Still other Examples are available from http://www.biology.duke.edu/fungi/mycolab/primers.htm.

As intended herein the primers to be used may be unmodified or modified nucleic acids, in particular DNA. Where the primers are modified nucleic acids they can notably be labelled nucleic acids, in particular fluorescently labelled nucleic acids.

Where TRFLP is used, the one of skill in the art knows how to design primers and to select restriction enzymes so that the nucleic acid generated and fragmented by the enzyme presents a fragment length that can be detected. In this regard, it should be noted that detection of a fragment is dependant on the instrumentation and detection techniques used, but in general detection is possible for sequences featuring at least 15 nucleotides. Besides, the length of the fragment should preferably be such that it is distinguishable from fragment lengths generated by the same primer pair/enzyme in other fungi (in general, fragment lengths differing by more than 1, and preferably more than 2, nucleotides are distinguished by current techniques). Numerous databases and tools are available to one of skill in the art for selecting primers and restriction enzymes, such as the REBASE database.

In the case where nucleic acids according to the invention include nucleotides featuring a "wobble" position, that is to say they include nucleotides that may be selected from among two or more possible nucleotides, one of skill in the art knows that it is usually advantageous to use a mixture of primers such that all of the nucleotide possibilities are represented.

Thus, by way of example, each one of the nucleic acids represented by GTGARTCATCGAAWYTTTGAACGCA (SEQ ID NO: 2), are as follows:
GTGAATCATCGAAACTTTGAACGCA (SEQ ID NO: 3);
GTGAGTCATCGAAACTTTGAACGCA (SEQ ID NO: 4);
GTGAATCATCGAATCTTTGAACGCA (SEQ ID NO: 5);
GTGAGTCATCGAATCTTTGAACGCA (SEQ ID NO: 6);
GTGAATCATCGAAATTTTGAACGCA (SEQ ID NO: 7);
GTGAGTCATCGAAATTTTGAACGCA (SEQ ID NO: 8);
GTGAATCATCGAATTTTTGAACGCA (SEQ ID NO: 9);
GTGAGTCATCGAATTTTTGAACGCA (SEQ ID NO: 10);
GTGAATCATCGAAACTTTGAACGCA (SEQ ID NO: 11);
GTGAGTCATCGAAACTTTGAACGCA (SEQ ID NO: 12);
GTGAATCATCGAATCTTTGAACGCA (SEQ ID NO: 13);
GTGAGTCATCGAATCTTTGAACGCA (SEQ ID NO: 14);
GTGAATCATCGAAATTTTGAACGCA (SEQ ID NO: 15);
GTGAGTCATCGAAATTTTGAACGCA (SEQ ID NO: 16);
GTGAATCATCGAATTTTTGAACGCA (SEQ ID NO: 17);
GTGAGTCATCGAATTTTTGAACGCA (SEQ ID NO: 18).

As intended herein, it is to be understood that the invention aims at the detection of nucleic acids of fungi of any type, however it is preferred that the fungus is a plant pathogenic fungus, in particular a turfgrass pathogenic fungus, such as a fungus selected from the group constituted of *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

As intended herein the sample in which nucleic acids are to be detected can be of any type of organic substrate liable to contain nucleic acids from fungi. However, it is preferred that the sample be a turfgrass sample or a soil sample.

Where the sample is a turfgrass sample, it can be a sample obtained from the turfgrass as a whole or from or a sample of a part of the turfgrass, such as the root.

Where the sample is a soil sample, it is preferably taken directly under the diseased turfgrass or in the vicinity of the diseased turgrass.

The sample can be obtained directly from turfgrass or soil, or be obtained after treatment steps, such as grinding or extraction, in particular nucleic acid extraction, steps.

As intended herein, any diseased turfgrass can be subjected the use or methods as defined above. Preferred turfgrasses to be considered within the frame of the present invention are notably described in http://www.ars-grin.gov/cgi-bin/npgs/html/index.pl, from the Germplasm Resources Information Network, National Germplasm Resources Laboratory, Beltsville, Maryland, or in the Compendium of Turgrass Diseases, Third Edition (2005) by the American Phytopathological Society. Most preferably, the diseased turfgrass according to the invention is selected from the group consisting of the *Festaceae, Aveneae, Triticeae, Chlorideae, Zoysieae, Paniceae* and *Andropogoneae Tribe.*

### EXAMPLE

The following turfgrass pathogenic fungus species were sequenced by the inventors: *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

Briefly, the well-known sequencing method developed by Fred Sanger - chain termination method - was used using Applied Biosystem BigDye® Terminator Cycle Sequencing v1 or v3.1 chemistry on an Applied Biosystems Genetic Analyzer 3130. The sequences were gathered by using the ITS1 forward primer (ITS1-F, TCCGTAGGTGAACCTGCGG, SEQ ID NO: 39).

The obtained sequences are represented by SEQ ID NO: 19 to 37.

From these sequences a particular consensus sequence could be determined by the inventors:
CATCGATGAAGAACGCWGCRAAHTGCGATAMGTARTGYGAATTGCAGRATTC AGTGARTCATCGAAWYTTTGAACGCAYMTTGCRC (SEQ ID NO: 1)

The discriminative potential of this region was then evidenced by *in silico* Terminal Restriction Fragment Length Polymorphism (TRFLP or T-RFLP) by using the primer ITSOMYAr (TGCGTTCAAARWTTCGATGAYTCAC, SEQ ID NO: 38, the complementary of SEQ ID NO: 2), which hybridizes to the above consensus sequence, in association with the above ITS1-F primer.

The applied enzymes for the T-RFLP analysis were *Taq*I (T^{▼}CG_{▲}A) and *Tsp*509I (^{▼}AATT_{▲}).

The following tables show the PCR fragment length of both primer combination ITS1-F/ITS4 (full length ITS) (ITS4, TCCTCCGCTTATTGATATGC, SEQ ID NO: 40) and ITS1/ITSOMYAr (partial ITS length) (Table 1), as well as the corresponding *in silico* and *in vitro* digested partial ITS (ITS1/ITSOMYAr) fragments by *Taq*I (Table 2) and *Tsp*509I (Table 3) (mean and standard deviation (StDev) were calculated from triplicate measurements of two independent PCR assays).

**Table 1**

| | **Fragments [bps]** | |
|---|---|---|
| **Fungal name** | **Full ITS length ITS1 /ITS4** | **Partial ITS length ITS1 /ITSOMYAr** |
| *Ascochyta phleina* | 588 | 326 |
| *Curvularia affinis* | 584 | 316 |
| *Glomerella graminicola* | 587 | *320* |
| *Thanatephorus cucumeris* | 710 | *360* |
| *Pythium ultimum* | 914 | *360* |
| *Gaeumannomyces graminis* | 558 | *280* |
| *Marasmius oreades* | 672 | 355 |
| *Corticium fuciforme* | 716 | 353 |
| *Phytophthora nicotianae* | 892 | 367 |
| *Fusarium culmorum* | 545 | 286 |
| *Bipolaris sorokiniana* | 586 | 309 |
| *Microdochium nivale* | 556 | 283 |
| *Rhizoctonia cerealis* | 684 | 348 |
| *Pythium graminicola* | 872 | 319 |
| *Rhynchosporium secalis* | 627 | 371 |
| *Sclerotinia homoeocarpa* | 586 | *320* |
| *Typhula incarnata* | 825 | 410 |
| *Ustilago striiformis* | 780 | *380* |
| *Septoria macropoda* | 540 | 277 |

**Table 2**

| | ***TaqI fragments [bps]*** | | |
|---|---|---|---|
| | ***in-silico*** | **measured** | |
| **Fungal name** | | **mean** | **StDev** |
| *Ascochyta phleina* | 252 | 247.0 | 0.022 |
| *Curvularia affinis* | 242 | 236.4 | 0.048 |
| *Glomerella graminicola* | 246 | 239.3 | 0.113 |
| *Thanatephorus cucumeris* | *286* | 284.4 | 0.123 |
| *Pythium ultimum* | *286* | 284.0 | 0.043 |
| *Gaeumannomyces graminis* | 206 | 198.9 | 0.030 |
| *Marasmius oreades* | 281 | 279.6 | 0.057 |
| *Corticium fuciforme* | 279 | 275.7 | 0.054 |
| *Phytophthora nicotianae* | 293 | 290.9 | 0.056 |
| *Fusarium culmorum* | 212 | 207.9 | 0.033 |
| *Bipolaris sorokiniana* | 235 | 230.2 | 0.054 |
| *Microdochium nivale* | 209 | 206.5 | 0.247 |
| *Rhizoctonia cerealis* | 71 | 67.7 | 0.049 |
| *Pythium graminicola* | 245 | 242.8 | 0.033 |
| *Rhynchosporium secalis* | 113 | 108.5 | 0.043 |
| *Sclerotinia homoeocarpa* | 102 | 98.1 | 0.043 |
| *Typhula incarnata* | 32 | 29.9 | 0.182 |
| *Ustilago striiformis* | 31 | 29.6 | 0.130 |
| *Septoria macropoda* | 162 | 147.2 | 0.161 |

**Table 3**

| | ***Tsp509I fragments [bps]*** | | |
|---|---|---|---|
| | ***in-silico*** | **measured** | |
| **Fungal name** | | **mean** | **StDev** |
| *Ascochyta phleina* | 167 | 163.5 | 0.041 |
| *Curvularia affinis* | 278 | 272.1 | 0.044 |
| *Glomerella graminicola* | 207 | 201.2 | 0.034 |
| *Thanatephorus cucumeris* | *34* | 32.3 | 0.091 |
| *Pythium ultimum* | 322 | 321.4 | 0.040 |
| *Gaeumannomyces graminis* | 134 | 125.7 | 0.096 |
| *Marasmius oreades* | 225 | 223.9 | 0.056 |
| *Corticium fuciforme* | 315 | 312.8 | 0.042 |
| *Phytophthora nicotianae* | 108 | 104.1 | 0.026 |
| *Fusarium culmorum* | 248 | 243.3 | 0.029 |
| *Bipolaris sorokiniana* | 193 | 189.0 | 0.032 |
| *Microdochium nivale* | 137 | 133.5 | 0.015 |
| *Rhizoctonia cerealis* | 310 | 307.6 | 0.032 |
| *Pythium graminicola* | 64 | 58.6 | 0.048 |
| *Rhynchosporium secalis* | 102 | 329.7 | 0.030 |
| *Sclerotinia homoeocarpa* | 175 | 172.0 | 0.066 |
| *Typhula incarnata* | 34 | 31.9 | 0.112 |
| *Ustilago striiformis* | 143 | 140.7 | 0.013 |
| *Septoria macropoda* | 239 | 222.4 | 0.169 |

In general, since the length of the fragments yielded by the ITS1/ITSOMYAr combination, either in AFLP or in T-RFLP, is usually smaller than the corresponding fragment obtained using the ITS1/ITS4 combination, the fragments can be separated with a higher resolution.

Furthermore, it can be seen that the ITS1/ITSOMYAr combination provides, either in AFLP or in T-RFLP, a good alternative to the ITS1/ITS4 combination in AFLP since it notably enables to discriminate between species which discrimination was either impossible or very difficult to carry out with the ITS1/ITS4 combination (*e*.*g*. *Ascochyta phleina, Curvularia affinis, Glomerella graminicola, Bipolaris sorokiniana, Sclerotinia homoeocarpa).*

## Claims

1. The use of at least one nucleic acid comprising or consisting of:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
R represents A or G
Y represents C or T
M represents A or C
W represents A or T
H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii);
for the detection of nucleic acids from one or more fungi in a sample.

2. The use according to claim 1, of at least one nucleic acid comprising or consisting of:
(i) GTGARTCATCGAAWYTTTGAACGCA (SEQ ID NO: 2), wherein:
R represents A or G
Y represents C or T
W represents A or T
(ii) a portion of SEQ ID NO: 2, provided said nucleic acid binds under stringent conditions to a nucleic acid comprising or consisting of the complementary sequence of SEQ ID NO: 1, or
(iii) complementary sequences of (i) and (ii).

3. The use according to claim 1 or 2, wherein the fungus is selected from the group constituted of *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

4. The use according to any of claims 1 to 3, wherein the sample is a turfgrass or a soil sample.

5. The use according to claim 4, wherein the sample is a turfgrass root sample.

6. The use according to claim 4 or 5, wherein the turfgrass is selected from the group

7. The use according to any of claims 1 to 6, wherein the at least one nucleic acid is used as a primer in a nucleic acid amplification-based detection method.

8. The use according to claim 7, wherein the nucleic acid amplification-based method is Amplification Fragment Length Polymorphism (AFLP) or Terminal Restriction Fragment Length Polymorphism (T-RFLP).

9. The use according to claim 7 or 8, wherein the at least one nucleic acid is used in association with at least one other primer which targets the 18S rDNA/ITS1 region or the ITS2/28S rDNA region.

10. The use according to claim ç, wherein the other primer is selected from the list constituted of SEQ ID NO: 39 to 42.

11. A method for treating a diseased turfgrass, which comprises the steps of:
a) detecting the absence or the presence of nucleic acids from at least one pathogenic fungus in a sample of soil from in which the diseased turfgrass is growing, or in a sample of the diseased turfgrass, with at least one nucleic acid as defined in claim 1 or 2;
b) If nucleic acids from one or more pathogenic fungi have been detected in step a), selecting one or more antifungal agents which target the one or more pathogenic fungi from which nucleic acids have been detected;
c) Applying the selected one or more antifungal agents of step b) to the diseased turfgrass.

12. The method according to claim 11, wherein the fungus is selected from the group constituted of *Ascochyta phleina, Curvularia affinis, Glomerella graminicola, Thanatephorus cucumeris, Pythium ultimum, Gaeumannomyces graminis, Marasmius oreades, Corticium fuciforme, Phytophthora nicotianae. Fusarium culmorum, Bipolaris sorokiniana, Microdochium nivale, Rhizoctonia cerealis, Pythium graminicola, Rhynchosporium secalis, Sclerotinia homoeocarpa, Typhula incarnate, Ustilago striiformis, Septoria macropoda.*

13. The method according to claim 11 or 12, wherein the diseased turfgrass is selected from the group consisting of the *Festaceae, Aveneae, Triticeae, Chlorideae, Zoysieae, Paniceae and Andropogoneae* Tribe*.*

14. The method according to any of claims 11 to 13, wherein the nucleic acids from the at least one pathogenic fungi are detected by a nucleic acid amplification method with the at least one nucleic acid as defined in claim 1 or 2 as a primer.

15. The method according to claim 14, wherein the nucleic acid amplification method is Amplification Fragment Length Polymorphism (AFLP) or Terminal Restriction Fragment Length Polymorphism (T-RFLP).

16. The method according to claim 14 or 15, wherein the at least one nucleic acid as defined in claim 1 or 2 is used, in association with at least one other primer which targets 18S rDNA/ITS1 region or the ITS2/28S rDNA region.

17. The method according to claim 16, wherein the other primer is selected from the list constituted of SEQ ID NO: 39 to 42.

18. A kit for the detection of fungi, comprising each one of the nucleic acids represented by:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
R represents A or G
Y represents C or T
M represents A or C
W represents A or T
H represents A or C or T
(ii) a portion of SEQ ID NO: 1, provided said nucleic acid binds under stringent conditions to a nucleic acid comprising or consisting of the complementary sequence of SEQ ID NO: 1, or
(iii) complementary sequences of (i) and (ii).

19. The kit according to claim 18, comprising the nucleic acids represented by the following sequences:
GTGAATCATCGAAACTTTGAACGCA (SEQ ID NO: 3);
GTGAGTCATCGAAACTTTGAACGCA (SEQ ID NO: 4);
GTGAATCATCGAATCTTTGAACGCA (SEQ ID NO: 5);
GTGAGTCATCGAATCTTTGAACGCA (SEQ ID NO: 6);
GTGAATCATCGAAATTTTGAACGCA (SEQ ID NO: 7);
GTGAGTCATCGAAATTTTGAACGCA (SEQ ID NO: 8);
GTGAATCATCGAATTTTTGAACGCA (SEQ ID NO: 9);
GTGAGTCATCGAATTTTTGAACGCA (SEQ ID NO: 10);
GTGAATCATCGAAACTTTGAACGCA (SEQ ID NO: 11);
GTGAGTCATCGAAACTTTGAACGCA (SEQ ID NO: 12);
GTGAATCATCGAATCTTTGAACGCA (SEQ ID NO: 13);
GTGAGTCATCGAATCTTTGAACGCA (SEQ ID NO: 14);
GTGAATCATCGAAATTTTGAACGCA (SEQ ID NO: 15);
GTGAGTCATCGAAATTTTGAACGCA (SEQ ID NO: 16);
GTGAATCATCGAATTTTTGAACGCA (SEQ ID NO: 17);
GTGAGTCATCGAATTTTTGAACGCA (SEQ ID NO: 18);
or their complementary sequences.

20. A nucleic acid comprising or consisting of:
(i) 
CATCGATGAAGAACGCWGCRAAHTGCGATAMGTARTGYGAATTGCAGR
ATTCAGTGARTCATCGAAWYTTTGAACGCAYMTTGCRC (SEQ ID NO: 1),
 wherein:
R represents A or G
Y represents C or T
M represents A or C
W represents A or T
H represents A or C or T
(ii) a portion of SEQ ID NO: 1, provided the said nucleic acid binds under stringent conditions to a nucleic acid comprising or consisting of the complementary sequence of SEQ ID NO: 1, or
(iii) complementary sequences of (i) and (ii).

21. A nucleic acid according to claim 20, comprising or consisting of a sequence selected from the group consisting of SEQ ID NO: 1 to 38, or complementary sequences thereof.


SEQUENCE LISTING

<110>  Omya Development AG

<120>  NUCLEIC ACIDS AND METHODS FOR DETECTING TURFGRASS PATHOGENIC
       FUNGI

<130>  BET08P0292

<160>  42

<170>  PatentIn version 3.4

<210>  1
<211>  86
<212>  DNA
<213>  Artificial sequence

<220>
<223>  Fungal consensus sequence

<400>  1
catcgatgaa gaacgcwgcr aahtgcgata mgtartgyga attgcagrat tcagtgartc      60

atcgaawytt tgaacgcaym ttgcrc                                           86


<210>  2
<211>  25
<212>  DNA
<213>  Artificial sequence

<220>
<223>  Degenerated PCR primer for fungal detection

<400>  2
gtgartcatc gaawytttga acgca                                            25


<210>  3
<211>  25
<212>  DNA
<213>  Artificial sequence

<220>
<223>  PCR primer for fungal detection

<400>  3
gtgaatcatc gaaactttga acgca                                            25


<210>  4
<211>  25
<212>  DNA
<213>  Artificial sequence

<220>
<223>  PCR primer for fungal detection

<400>  4
gtgagtcatc gaaactttga acgca                                            25

<210>  5
<211>  25
<212>  DNA
<213>  Artificial sequence

<220>
<223>  PCR primer for fungal detection

<400>  5
gtgaatcatc gaatctttga acgca                                              25


<210>  6
<211>  25
<212>  DNA
<213>  Artificial sequence

<220>
<223>  PCR primer for fungal detection

<400>  6
gtgagtcatc gaatctttga acgca                                              25


<210>  7
<211>  25
<212>  DNA
<213>  Artificial sequence

<220>
<223>  PCR primer for fungal detection

<400>  7
gtgaatcatc gaaattttga acgca                                              25


<210>  8
<211>  25
<212>  DNA
<213>  Artificial sequence

<220>
<223>  PCR primer for fungal detection

<400>  8
gtgagtcatc gaaattttga acgca                                              25


<210>  9
<211>  25
<212>  DNA
<213>  Artificial sequence

<220>
<223>  PCR primer for fungal detection

<400>  9
gtgaatcatc gaattttga acgca                                              25

<210>  10
<211>  25
<212>  DNA
<213>  Artificial sequence

<220>
<223>  PCR primer for fungal detection

<400>  10
gtgagtcatc gaatttttga acgca                                              25


<210>  11
<211>  25
<212>  DNA
<213>  Artificial sequence

<220>
<223>  PCR primer for fungal detection

<400>  11
gtgaatcatc gaaactttga acgca                                              25


<210>  12
<211>  25
<212>  DNA
<213>  Artificial sequence

<220>
<223>  PCR primer for fungal detection

<400>  12
gtgagtcatc gaaactttga acgca                                              25


<210>  13
<211>  25
<212>  DNA
<213>  Artificial sequence

<220>
<223>  PCR primer for fungal detection

<400>  13
gtgaatcatc gaatctttga acgca                                              25


<210>  14
<211>  25
<212>  DNA
<213>  Artificial sequence

<220>
<223>  PCR primer for fungal detection

<400>  14
gtgagtcatc gaatctttga acgca                                              25

<210> 15
<211> 25
<212> DNA
<213> Artificial sequence

<220>
<223> PCR primer for fungal detection

<400> 15
gtgaatcatc gaaattttga acgca 25


<210> 16
<211> 25
<212> DNA
<213> Artificial sequence

<220>
<223> PCR primer for fungal detection

<400> 16
gtgagtcatc gaaattttga acgca 25


<210> 17
<211> 25
<212> DNA
<213> Artificial sequence

<220>
<223> PCR primer for fungal detection

<400> 17
gtgaatcatc gaatttttga acgca 25


<210> 18
<211> 25
<212> DNA
<213> Artificial sequence

<220>
<223> PCR primer for fungal detection

<400> 18
gtgagtcatc gaatttttga acgca 25


<210> 19
<211> 589
<212> DNA
<213> Ascochyta phleina

<400> 19
tccgtaggtg aacctgcgga aggatcatta cactcagtag tttactactg taaaggaggc 60

cgtcagtctg tatagcttca tcgctgacga gcagctggtc tcttttatcc acccttgtct 120

tttgcgtacc catgtttcct cggcaggctt gcctgccgac tggacaaatt tataaccttt 180

ttaattttca atcagcgtct gaaaaactta ataattacaa ctttcaacaa cggatctctt 240

ggttctggca tcgatgaaga acgcagcgaa atgcgataag tagtgtgaat tgcagaattc 300

agtgaatcat cgaatctttg aacgcacatt gcgccccttg gtattccatg gggcatgcct 360

gttcgagcgt catttgtacc ctcaagcttt gcttggtgtt gggtgtttgt cctctccctg 420

gtgtttggac tcgccttaaa acaattggca gccagtgttt tggtattgaa gcgcagcaca 480

agtcgcgatt ctaacctagt aacacttgcg tccacaagcc ttttttcact tttgacctcg 540

gatcaggtag ggatacccgc tgaacttaag catatcaata agcggagga 589

<210> 20
<211> 584
<212> DNA
<213> Curvularia affinis

<400> 20
tccgtaggtg aacctgcgga gggatcatta cacaataaac atatgaaggc tgcaccgcca 60

gttggcggca aggctggagt attttattac ccttgtcttt tgcgcacttg ttgtttcctg 120

ggcgggttcg cccgccacca ggaccacatg ataaaccttt tttatgcagt tgcaatcagc 180

gtcagtacaa caaatgtaaa tcatttacaa ctttcaacaa cggatctctt ggttctggca 240

tcgatgaaga acgcagcgaa atgcgatacg tagtgtgaat tgcagaattc agtgaatcat 300

cgaatctttg aacgcacatt gcgccctttg gtattccaaa gggcatgcct gttcgagcgt 360

catttgtacc ctcaagcttt gcttggtgtt gggcgttttg tctttggttg ccaaagactc 420

gccttaaaac gattggcagc cggcctactg gtttcgcagc gcagcacatt tttgcgcttg 480

caatcagcaa aagaggacgg cactccatca agactcttta tcacttttga cctcggatca 540

ggtagggata cccgctgaac ttaagcatat caataagcgg agga 584

<210> 21
<211> 587
<212> DNA
<213> Glomerella graminicola

<400> 21
tccgtaggtg aacctgcgga gggatcatta ctgagttacc gctctataac cctttgtgaa 60

catacctaac cgttgcttcg gcgggttagg gggtcccctc tccgggggac gccctcccgg 120

ccgggcccca ctgcggggct cggcgcccgc cggaggataa ccaaactctg atttaacgac 180

gtctcttctg agtggcacaa gcaaataatt aaaactttta acaacggatc tcttggttct 240

ggcatcgatg aagaacgcag cgaaatgcga taagtaatgt gaattgcaga attcagtgaa 300

tcatcgaatc tttgaacgca cattgcgccc gccagcattc tggcgggcat gcctgttcga 360

gcgtcatttc aaccctcaag ctccgcttgg tgttggggcc ctacggcgta cgtcgtaggc 420

ccttaaaggt agtggcggac cctcccggag cctcctttgc gtagtaacta acgtctcgca    480

tcgggatccg gagggactct tgccgtaaaa cccccaactt tttaactggt tgacctcgga    540

tcaggtagga atacccgctg aacttaagca tatcaataag cggagga                  587


<210>  22
<211>  710
<212>  DNA
<213>  Thanatephorus cucumeris

<400>  22
tccgtaggtg aacctgcgga aggatcatta ttgaatttta ttaatgagga gttgagttgt     60

tgctggcctt ttctacctta atttggcagg agggggcatg tgcacacctt ctcttttatc    120

catcacaccc cctgtgcact tgtgagacag caatagttgg tggatttaat tccatccatt    180

tgctgtctac ttaatttaca cacactctac ttaatttaaa ctgaatgtaa ttgatgtaac    240

gcatctaata ctaagtttca acaacggatc tcttggctct cgcatcgatg aagaacgcag    300

cgaaatgcga taagtaatgt gaattgcaga attcagtgaa tcatcgaatc tttgaacgca    360

ccttgcgctc cttggtattc cttggagcat gcctgtttga gtatcatgaa atcttcaaag    420

taaacctttt gttaattcaa ttggtctttt ttactttggt tttggaggat cttattgcag    480

cttcacacct gctcctcttt gtgcattagc tggatctcag tgttatgctt ggttccactc    540

ggcgtgataa gttatctatc gctgaggaca cccgtaaaaa aggtggccaa ggtaaatgca    600

gatgaaccgc ttctaatagt ccattgactt ggacaatatt ctattttatg atctgatctc    660

aaatcaggta ggactacccg ctgaacttaa gcatatcaat aagcggagga                710


<210>  23
<211>  914
<212>  DNA
<213>  Pythium ultimum

<400>  23
tccgtaggtg aacctgcgga aggatcatta ccacacttta aaaaactgtc cacgtgaact     60

gtaagcaagt ctagcgctgt gactgagctg gtgttttcat ttttggacac tggaacggga    120

gtcagcagga cgaaggttgg tctgttgtaa tgcaagttat gatggactag ctgatgaact    180

tttgttttta aacccttacc taaatactga tttatactgt ggggacgaaa gtccttgctt    240

ttactagata acaactttca gcagtggatg tctaggctcg cacatcgatg aagaacgctg    300

cgaactgcga tacgtaatgc gaattgcaga attcagtgag tcatcgaaat tttgaacgca    360

tattgcactt tcgggttatg cctggaagta tgtctgtatc agtgtccgta aatcaaactt    420

gcctttcttt ttccgtgtag tcagggatgg aatgtgcaga tgtgaagtgt ctcgcatggt    480

tgcgttcgtt ttttcgatcg agaatctgtc gagtcctttt aaatggacac ggtcttttct    540

atggtttcta tgaagtgtaa tggttggaag gcagtgattt tcggattgct ggcggctttt 600

ggcgacttcg gtatgaacgt atggagacta gctcaattcg tggtatgtta ggcttcggct 660

cgacaatgtt gcgtaattgt gtgtggtctt tgtttgtgcc ttgaggtgta ctagaggttg 720

tcggtttgaa ccgtaagtga ttgtttagta gagcattttc acgatgtatg gagacgctgc 780

atttagttgc gtagagagat tgatttggga aattttgtat cattgtcaat tgcaagattg 840

tgtatggtat ctcaattgga cctggtatca gacaagacta cccgctgaac ttaagcatat 900

caataagcgg agga 914

<210> 24
<211> 558
<212> DNA
<213> Gaeumannomyces graminis

<400> 24
tccgtaggtg aacctgcgga gggatcatta cagagttgaa aaactccaac ccctgtgaac 60

atacctttac tgttgcttcg gcggacgatg gccccccccg ggggccggac gccgccggag 120

gttacaaacc ctgaatttta gtgtatctct gagtataaaa ccaaataatt aaaactttca 180

acaacggatc tcttggttct ggcatcgatg aagaacgcag cgaaatgcga taagtaatgt 240

gaattgcaga attcagtgaa tcatcgaatc tttgaacgca cattgcgccc gccggtattc 300

cggcgggcat gcctgtccga gcgtcatttc accactcaag cccagcttgg tgttggggca 360

cccggccgcc cggcggtcgg ggcccccaag aacatcggcg gtctcgccag gaccctgaac 420

gcagtaactc gcggtaaaac gcgcttcgtt cggaggcttc ccggcgggct ccagccgcta 480

aaccccctaa acttcttagg ttgacctcgg atcaggtagg aatacccgct gaacttaagc 540

atatcaataa gcggagga 558

<210> 25
<211> 672
<212> DNA
<213> Marasmius oreades

<400> 25
tccgtaggtg aacctgcgga aggatcatta ttgaaacatt gtaaaggaag gttgagctgg 60

ctcttcacgg gcatgtgctc gcctttcttt caatcttcat ccacctgtgc actttttgta 120

gggagctttg agaatgggac ctctcgcggg gttcctagta ttgggctctc tatgtcttca 180

cacactcttg aatgtatgtc gttgaatgtc ttttacaggg acttaattga ccctttaaaa 240

actatacaac tttcagcaac ggatctcttg gctctcgcat cgatgaagaa cgcagcgaaa 300

tgcgataagt aatgtgaatt gcagaattca gtgaatcatc gaatctttga acgcaccttg 360

cgcctcttgg tattccgaga ggcatgcctg tttgagtgtc attaaattct caacttcaaa 420

agcttttgtt tttgaagctt ggatgtggag gctttgctgg ctcttctaga gtcggctcct 480

ctgaaatgca ttagtggaaa ctgtttgcaa tccgcattgg tgtgataatt atctacgctt 540

gtgtgtggct gcagctcttt acgagtttag tatccgcttc aaaccgtcct aagttactgg 600

acaacttgaa ccttttgacc tcaaatcagg taggactacc cgctgaactt aagcatatca 660

ataagcggag ga 672

<210> 26
<211> 716
<212> DNA
<213> Corticium fuciforme

<400> 26
tccgtaggtg aacctgcgga aggatcatta acgagtttta aaagagttgt agctggccct 60

ctggggtatg tgcacgctct actcatccac atacacctgt gcacatagat agtctttttt 120

ttgagaaggg ggagaaaaag tagccctttg ggtgaaaagt ttcccccctc ttcagaaagc 180

tattcttttt atacacatac actttagtta agaatgtata tattgctata aaacgcatta 240

aatataactt tcaacaacgg atctcttggc tctcgcatcg atgaagaacg cagcgaaatg 300

cgataagtaa tgtgaattgc agaattcagt gaatcatcga atctttgaac gcaccttgcg 360

ctccttggta ttccgaggag catgcctgtt tgagtgtcat gaatatctca actctcaaag 420

ttctgtaatg gatcattgag agcttggact ttggaggctt gctggtcatc atgtatcagc 480

tcctcttaaa tgaattagct ctggattacg atgtagttta tctacggtgt gatacatgtc 540

tacgctatta gataactacg aacgttacaa ttcctccatt tctggggttt gcgtgactag 600

taaatacaaa gcttctaatt gtctttcggg acaaaacctg atttcattat cttgacatct 660

gacctcaaat caggtaggac tacccgctga acttaagcat atcaataagc ggagga 716

<210> 27
<211> 892
<212> DNA
<213> Phytophthora nicotianae

<400> 27
tccgtaggtg aacctgcgga aggatcatta ccacacctaa aaaactttcc acgtgaaccg 60

tttcaaccca atagttgggg gtcttatttg gcggcggctg ctggcttaat tgttggcggc 120

tgctgctgag tgagccctat caaaaaaaag gcgaacgttt gggcttcggc ctgatttagt 180

agtctttttt tcttttaaac ccattcctta atactgaata tactgtgggg acgaaagtct 240

ctgcttttaa ctagatagca actttcagca gtggatgtct aggctcgcac atcgatgaag 300

aacgctgcga actgcgatac gtaatgcgaa ttgcaggatt cagtgagtca tcgaaatttt 360

gaacgcatat tgcacttccg ggttagtcct ggaagtatgc ctgtatcagt gtccgtacat    420

taaacttgac tttcttcctt ccgtgtagtc ggtggaggag atgtcagatg tgaagtgtct    480

tgcgattggt cttcggaccg gctgcgagtc cttttaaatg tactaaactg aacttctctt    540

tgctcgaaaa gtggtggcgt tgctggttgt gaaggctgct attgtggcaa attggcgact    600

ggtttgtctg ctgcggcgtt aatgggagag tgttcgattc gtggtatggt tggcttcggc    660

tgaacaatgc acttattgga cgtttttcct gctgtggcgt gatggactgg tgaaccatag    720

ctcggtggct tggcttttga attggctttg ctgttgcgaa gtagggtggc agcttcggtt    780

gtcgagggtc gatccatttg ggaacttaat gtgtacttcg gtatgcatct caattggacc    840

tgatatcagg caagattacc cgctgaactt aagcatatca ataagcggag ga          892

<210>  28
<211>  545
<212>  DNA
<213>  Fusarium culmorum

<400>  28
tccgtaggtg aacctgcgga gggatcatta ccgagtttac aactcccaaa cccctgtgaa     60

cataccttat gttgcctcgg cggatcagcc cgcgccccgt aaaaagggac ggcccgccgc    120

aggaacccta aactctgttt ttagtggaac ttctgagtat aaaaaacaaa taaatcaaaa    180

cttccaacaa cggatctctt ggttctggca tcgatgaaga acgcagcaaa atgcgataag    240

taatgtgaat tgcagaattc agtgaatcat cgaatctttg aacgcacatt gcgcccgcca    300

gtattctggc gggcatgcct gttcgagcgt catttcaacc ctcaagccca gcttggtgtt    360

gggagctgca gtcctgctgc actccccaaa tacattggcg gtcacgtcga gcttccatag    420

cgtagtaatt tacatatcgt tactggtaat cgtcgcggcc acgccgttaa accccaactt    480

ctgaatgttg acctcggatc aggtaggaat acccgctgaa cttaagcata tcaataagcg    540

gagga                                                                545

<210>  29
<211>  586
<212>  DNA
<213>  Bipolaris sorokiniana

<400>  29
tccgtaggtg aacctgcgga gggatcatta cacaacaaaa tatgaaggcc tggcttcgcg     60

gccggctgaa atattttttt cacccatgtc ttttgcgcac ttgttgtttc ctgggcgggt    120

tcgcccgcca ccaggaccaa accataaacc ttttttttat gcagttgcaa tcagcgtcag    180

taaaaacaat gtaattatta caactttcaa caacggatct cttggtcctg gcatcgatga    240

agaacgcagc gaaatgcgat acgtagtgtg aattgcagaa ttcagtgaat catcgaatct 300

ttgaacgcac attgcgccct ttggtattcc aaagggcatg cctgttcgag cgtcatttgt 360

accttcaagc tttgcttggt gttgggcgtt ttttgtctcc ctctttctgg gagactcgcc 420

ttaaaacgat tggcagccgg cctactggtt tcggagcgca gcacatattt tgcgctttgt 480

atcaggagaa aaggacggta atccatcaag actctacatt tttaactttt gacctcggat 540

caggtaggga tacccgctga acttaagcat atcaataagc ggagga 586

<210> 30
<211> 556
<212> DNA
<213> Microdochium nivale

<400> 30
tccgtaggtg aacctgcgga gggatcatta ctgagttttt aactctccaa accatgtgaa 60

cttaccactg ttgcctcggt ggatggtgct gtctctcggg acggtgccac cgccggtgga 120

ctacctaaac tctgttaatt tttgtcaatc tgaatcaaac taagaaataa gttaaaactt 180

tcaacaacgg atctcttggt tctggcatcg atgaagaacg cagcgaaatg cgataagtaa 240

tgtgaattgc agaattcagt gaatcatcga atctttgaac gcacattgcg cccattagta 300

ttctagtggg catgcctgtt cgagcgtcat ttcaaccctt aagcctagct tagtgttggg 360

agactgccta atacgcagct cctcaaaacc agtggcggag tcggttcgtg ctctgagcgt 420

agtaattttt tatctcgctt ctgcaagccg gactggcaac agccataaac cgcacccttc 480

gggggcactt tttaatggtt gacctcggat caggtaggaa tacccgctga acttaagcat 540

atcaataagc ggagga 556

<210> 31
<211> 684
<212> DNA
<213> Rhizoctonia cerealis

<400> 31
tccgtaggtg aacctgcgga aggatcatta gtgaatgaat gtagagtcgg ttgtagctgg 60

gtcttttaat cgaggccatg tgcacgcctt ctctttcatc cacacacacc tgtgcacctg 120

tttagacggt cgaaggaaaa agtctttctc gcgagagaga ggctggctcc ttttccgtcc 180

aatacataaa atcttatata tttaatcaga atgtaatcga tgtaaacgca tctataaact 240

aagtttcaac aacggatctc ttggctctcg catcgatgaa gaacgcagcg aaatgcgata 300

agtaatgtga attgcagaat tcagtgaatc atcgaatctt tgaacgcacc ttgcgctcct 360

tggtattcct cggagcacgc ctgtttgagt atcatgaaat tctcaaagca agtcttttgt 420

taattcaact ggcttttgtt ttggatttgg aggttttgca gattcacgtc tgctcctctt 480

aaatgcatta gctggatctc tataaaaccg gttccactcg gcgtgataag tatcactcgc    540

tgaggacact cttgaaaaag ggtggccgga ttcatggatg aaccgcttct aacggtctat    600

tagattagac aaacacactt tatgatctga tctcaaatca ggtgggacta cccgctgaac    660

ttaagcatat caataagcgg agga                                           684


<210>  32
<211>  872
<212>  DNA
<213>  Pythium graminicola

<400>  32
tccgtaggtg aacctgcgga aggatcatta ccacaccaaa aaactttcca cgtgaaccgt     60

tataattatg ttctgtgctt tccttcggga aggctgaacg aaggttgatc gtatgtatta    120

atttatgtgt ggtcttccga tgtcttttaa acccattact taatactgat ctatactccg    180

agaacgaaag tttttggttt taatccataa caactttcag cagtggatgt ctaggctcgc    240

acatcgatga agaacgctgc gaactgcgat acgtaatgcg aattgcagaa ttcagtgagt    300

catcgaaatt ttgaacgcac attgcacttt cgggatattc ctggaagtat gcttgtatca    360

gtgtccgtac atcaaacttg cctttctttt tttgtgtagt caaggagaga aatggccgat    420

tgtgaggtgt ctcgttgact cccttttcgg aggagaagac gcgagtccct ttaaatgtac    480

gttcgctctt tcttgtgtct gaggtgaagt gtgactttcg aacgcattga tctgtttgga    540

tcgttttgcg cgagtgggcg acttcggtta ggacgttaaa ggaagcaacc attattggcg    600

gtatgttaga cttcggtccg actttgcagc tgagagtgtg tagttttctg ttctttcctt    660

gaggtgtacc tgtttgtgtg aggcaatggt ctgagcaaat ggttattgtg tagtagaatt    720

ttgctgctct tggacgccct attcgtaggg taaagtagac aacaccattt tgggactagt    780

ctatgtatta atttatgtgg gcgatttttc aatttggacc tgatatcaag taagactacc    840

cgctgaactt aagcatatca ataagcggag ga                                  872


<210>  33
<211>  627
<212>  DNA
<213>  Rhynchosporium secalis

<400>  33
tccgtaggtg aacctgcgga aggatcatta atagagcaaa gaacagtcag cgccctagga     60

gaaatcccgg gggctaccct acttcggtgg ggtttagaga cgtcaggccg ctcgaagaag    120

cctggttcag acctccaccc ttgaataaac tacctttgtt gctttggcag gccgcccagc    180

gccagcggct tcggctgctg agtgcctgcc agaggaccac aactcttgtt tttagtgatg    240

tctgagtact atataatagt taaaactttc aacaacggat ctcttggttc tggcatcgat 300

gaagaacgca gcgaaatgcg ataagtaatg tgaattgcag aattcagtga atcatcgaat 360

ctttgaacgc acattgcgcc ctctggtatt ccggggggca tgcctgttcg agcgtcatta 420

taaccactca agctctcgct tggtattgga gttcgcgtcc tcgcggcccc taaaatcagt 480

ggcggtgcct gtcggctcta cgcgtagtaa tactcctcgc gattgagtcc ggctggtcta 540

cttgccaata acccccaaat ttttacaggt tgacctcgga tcaggtaggg atacccgctg 600

aacttaagca tatcaataag cggagga 627

<210> 34
<211> 586
<212> DNA
<213> Sclerotinia homoeocarpa

<400> 34
tccgtaggtg aacctgcgga aggatcatta ccgagttcac gccctcacgg gtagacctcc 60

aacccttgtg tatctctacc atgttgcttt ggcaggctgc tcgacccttc cggggacagc 120

ctcagcgccc tccggggccg gagagtcgcc tgccggagga aaatcacaac tctgaattgt 180

cagtgtcgtc tgagtgacta tctaatagtt aaaactttca acaacggatc tcttggttct 240

ggcatcgatg aagaacgcag cgaaatgcga taagtaatgt gaattgcaga attcagtgaa 300

tcatcgaatc tttgaacgca cattgcgccc cttggtattc cggggggcat gcctgttcga 360

gcgtcatttc aaccctcaag ctctctgctt ggtattgggc ctccgccggt cacacggcgg 420

gccttaaagt cagtggcggc gccgctgggt cctgaacgta gtaacacata cctctcgtta 480

cagggtcccc gcgcgctccc gccgtaaaac ccccctcatt ttctctggtt gacctcggat 540

caggtaggga tacccgctga acttaagcat atcaataagc ggagga 586

<210> 35
<211> 825
<212> DNA
<213> Typhula incarnate

<400> 35
tccgtaggtg aacctgcgga aggatcatta tcgaatttaa ggctttggtt gagctggcgc 60

ttcggtgcat gtgcttgcct tgtgctgtcc attcttcaac acctgtgcac actttgtagt 120

tgactctttt gtttatctgt tcatctctct cttgactccg gtctctgtga aggggtgcgt 180

ggcttttcga aagcaaggtc ctctatgtta ttattataca ccctttacaa aaaacaagtc 240

catagaacgt ccaatgtagg cgcagcgtaa aacactgttt gctgaaatta taaaacttat 300

acaactttta acaacggatc tcttggctct cgcatcgatg aagaacgcag cgaaatgcga 360

taagtaatgt gaattgcaga attcagtgaa tcatcgaatc tttgaacgca ccttgcgctc 420

cctggtattc cggggagcat gcctgtttga gtgtcattaa attctcaacc acactatgtt    480

tttattaacg tagttcttgt ggcttggatc ttggagtttg tgccggtaaa cctttagtta    540

ggttgttggc tcctctttaa atgcattagc tggaacctct ttgtggtgcc agactatggt    600

gtgataatta tctacgctgt ggtttgttgc gctgcgaatt taactatggg gttctgcttc    660

taatcgtccc tttcaaagga cagtatagag tgtggtgggg tgggggttgc tttcaagggg    720

ttcgcctctt gtgttacaat cttgcccttt acctcttaat tgacattttg acctcaaatc    780

aggtaggact acccgctgaa cttaagcata tcaataagcg gagga                    825


<210>  36
<211>  780
<212>  DNA
<213>  Ustilago striiformis

<400>  36
tccgtaggtg aacctgcgga tggatcattt cgatgaaaac ctttttttct tgaggtgtgg     60

ctcgcacctg tctaactaaa cttgagctac cttttttcaa cacggttgca tcggttggcc    120

tgtcaaacag tgcggcggcg tgaattttca cgtctgcttt ggctgggcga cggaccgaca    180

cttaatcaac acttttgata atctaggatt tgaatgataa aagttcattt ttacaatgaa    240

atcgactggt aatgcggtcg tctaattttt aaaaacaact tttggcaacg gatctcttgg    300

ttctcccatc gatgaagaac gcagcgaatt gcgataagta atgtgaattg cagaagtgaa    360

tcatcgaatc tttgaacgca ccttgcgctc ccggcagatc taatctgggg agcatgcctg    420

tttgagggcc gcgaattgtt tcgaacgaca actttttctc tctttttttt gaagagttgg    480

cggatcggta ttgaggtttt tgccattcac cgtggctccc tcgaaatgca ttagcgcatc    540

catttgatag gcaagacgga cgaaagctcg attttcgctc tctcttccct gccgggtttc    600

gataatatca ggacttcgga gaggttgaga tgggtaagag ctggacgcaa cggcttgctg    660

tttggagtgc ttctgaaacc cgcccatatc gagctttgcc tcggaaggga ctttaataat    720

tcatcggcct cagattggta ggactacccg ctgaacttaa gcatatcaat aagcggagga    780


<210>  37
<211>  540
<212>  DNA
<213>  Septoria macropoda

<400>  37
tccgtaggtg aacctgcgga gggatcatta ccgagtgggg gcctccgggt ccgatctcca     60

accctttgtg aacacatccc gttgcttcgg gggcgcccgg gccgggcgcc cccggaggac    120

catccaacac tgcatctttg cgtcggagtt tacgagtaaa tcgaaacaaa actttcaaca    180

acggatctct tggttctggc atcgatgaag aacgcagcga aatgcgataa gtaatgtgaa    240

ttgcagaatt cagtgaatca tcgaatcttt gaacgcacat tgcgccccct ggtattccgg    300

ggggcatgcc cgttcgagcg tcattacacc actccagcct cgctgggtat tgggcgtccg    360

cgggggagca atcccccgcg cgcctcaaag tctccggctg agcggtcccg tctcccagcg    420

ttgtggcatc acgtctcgcc gcggagtctc gggccctcac ggccgttaaa tcacacctca    480

ggttgacctc ggatcgggta gggatacccg ctgaacttaa gcatatcaat aagcggagga    540


<210>  38
<211>  25
<212>  DNA
<213>  Artificial sequence

<220>
<223>  Degenerated PCR primer for fungal detection

<400>  38
tgcgttcaaa rwttcgatga ytcac                                          25


<210>  39
<211>  19
<212>  DNA
<213>  Artificial sequence

<220>
<223>  ITS1-F primer

<400>  39
tccgtaggtg aacctgcgg                                                 19


<210>  40
<211>  20
<212>  DNA
<213>  Artificial sequence

<220>
<223>  ITS4 primer

<400>  40
tcctccgctt attgatatgc                                                20


<210>  41
<211>  22
<212>  DNA
<213>  Artificial sequence

<220>
<223>  ITS5 primer

<400>  41
ggaagtaaaa gtcgtaacaa gg                                             22

<210> 42
<211> 20
<212> DNA
<213> Artificial sequence

<220>
<223> SR6R primer

<400> 42
aagwaaaagt cgtaacaagg 20